# EUROPEAN PATENT APPLICATION

(11) **EP 4 365 186 A1**
(43) Date of publication of application: **08.05.2024**
(21) Application number: 22833564.2
(22) Date of filing: 28.06.2022
(51) Int. Cl.: C07K 7/06, C07K 7/08, C07K 14/00, A61K 38/00, A61P 3/10

(54) **PEPTIDE HAVING ANTIDIABETIC ACTIVITY AND USE THEREOF**

(30) Priority: 28.06.2021 KR 20210084286
(71) Applicant: Medi&Gene Inc., Seoul 02841 (KR)
(72) Inventor: SHIN, Min Jeong, Seoul 06285 (KR); JEONG, In Hyeok, Goyang-si Gyeonggi-do 10521 (KR)
(74) Representative: Maiwald GmbH
(86) International application number: PCT/KR2022/009198
(87) International publication number: WO 2023/277508

(57) **Abstract**

The present invention relates to a novel peptide having an antidiabetic activity. The peptide improves sugar metabolism and insulin resistance, inhibits amyotrophy while increasing muscle mass, and protects renal functions, and thus may be used for treating and relieving diabetes and diabetic complications.

## Description

### [Technical Field]

The present invention relates to a peptide having an antidiabetic activity and a pharmaceutical composition for treating diabetes mellitus, including the same.

### [Background Art]

Diabetes mellitus is a type of metabolic disease in which an amount of insulin secretion is insufficient or normal function is not achieved, is characterized by hyperglycemia, which increases a blood glucose concentration, and causes various symptoms and signs and causes glucose to be excreted in urine. Diabetes mellitus is classified into insulin-dependent diabetes (type 1 diabetes), non-insulin-dependent diabetes (type 2 diabetes) and malnutrition related diabetes mellitus (MRDM). Type 2 diabetes, which accounts for 90% or more of diabetic patients in South Korea, is a metabolic disease characterized by hyperglycemia, and is reported to occur due to a decrease in insulin secretion from pancreatic beta cells due to genetic, metabolic, and environmental factors or an increase in insulin resistance in peripheral tissues. In this regard, it is known that when body fat increases due to obesity, a symptom in which insulin sensitivity decreases is shown, and in particular, the accumulation of abdominal fat is associated with glucose intolerance. Moreover, it is known that in patients with type 2 diabetes, the more obese the patient is, the more severe insulin resistance becomes because obesity and insulin resistance are closely correlated. Further, diabetes mellitus induces complications such as angina pectoris, myocardial infarction, diabetic retinopathy, and diabetic nephropathy. Therefore, it is important to treat diabetes mellitus by not only controlling blood sugar but also managing complications.

### [Disclosure]

### [Technical Problem]

Under this background, the present inventors discovered a novel functional peptide and confirmed that this peptide has antidiabetic efficacy that improves insulin resistance and promotes sugar metabolism.

Therefore, an object of the present invention provides a peptide having an antidiabetic activity and a pharmaceutical composition for preventing or treating diabetes mellitus, including the same.

### [Technical Solution]

To achieve the above object, an aspect of the present invention provides a peptide consisting of an amino acid sequence selected from the group consisting of SEQ ID NOS: 1 to 14.

According to an exemplary embodiment of the present invention, the amino acid sequences of SEQ ID NOS: 1, 4, 6, 7, and 8 commonly include the sequence of SEQ ID NO: 1, and the amino acid sequences of SEQ ID NOS: 2, 4, 7, and 8 commonly include the sequence of SEQ ID NO: 2. In addition, the sequences of SEQ ID NOS: 3, 7, and 8 commonly include the sequence of SEQ ID NO: 3. The sequences of SEQ ID NOS: 5 and 6 commonly include the sequence of SEQ ID NO: 5.

The peptide disclosed in the present specification may include peptides with a sequence homology of 80% or more, 85% or more, 90% or more, 95% or more, 96% or more, 97% or more, 98% or more, or 99% or more. Furthermore, the peptide disclosed in the present specification may include a peptide in which one or more amino acids, two or more amino acids, three or more amino acids, four or more amino acids, five or more amino acids, six or more amino acids, or seven or more amino acids are changed from a peptide consisting of amino acid sequences selected from the group consisting of SEQ ID NOS: 1 to 8 or fragments thereof.

Meanwhile, a mouse sequence corresponding to an amino acid sequence selected from the group consisting of SEQ ID NOS; 1 to 8 is shown in the following Table 1.

**[Table 1]**

| SEQ ID NO: of human sequence | Peptide sequence (mouse) | SEQ ID NO: mouse sequence |
|---|---|---|
| 1 | YFR | - |
| 2 | EKAEEDR | 9 |
| 3 | REAGGAFGKR | - |
| 4 | EKAEEDRYFR | 10 |
| 5 | EKTKEQLAALRKHH | 11 |
| 6 | YFREKTKEQLAALRKHH | 12 |
| 7 | AGSIREAGGAFGKREKAEEDRYFR | 13 |
| 8 | MDTGAGSIREAGGAFGKREKAEEDRYFR | 14 |

As can be seen in Table 1, the sequence of SEQ ID NO: 1 is the same for humans and mice, and the sequence of SEQ ID NO: 1 is also included in the sequences of SEQ ID NOS: 10 and 12 to 14. Further, the sequence of SEQ ID NO: 9 is included in the sequences of SEQ ID NOS: 10, 13, and 14. The sequence of SEQ ID NO: 10 is included in the sequences of SEQ ID NOS: 13 and 14, and the sequence of SEQ ID NO: 11 is included in the sequence of SEQ ID NO: 12. Meanwhile, the sequence of SEQ ID NO: 3 is also the same for humans and mice, and is included in the sequences of SEQ ID NOS: 13 and 14. In an aspect of the present invention, the change in amino acid is a property that allows the physicochemical properties of a peptide to be altered. For example, the change in amino acid may be made to improve the thermal stability of the peptide, alter substrate specificity, change the optimal pH, and the like.

In the present specification, "amino acid" includes the 22 standard amino acids that are naturally incorporated into peptides, as well as D-isomers and modified amino acids thereof. Accordingly, in an aspect of the present invention, the peptide may be a peptide containing a D-amino acid. Meanwhile, in another aspect of the present invention, the peptide may include a non-standard amino acid that has been subjected to post-translational modification. Examples of the post-translational modification include phosphorylation, glycosylation, acylation (for example, including acetylation, myristoylation and palmitoylation), alkylation, carboxylation, hydroxylation, glycation, biotinylation, ubiquitinylation, changes in chemical properties (for example, beta-removal deimidation, or deamidation) and structural changes (for example, formation of disulfide bridges). In addition, examples of the post-translational modification include changes in amino acids, such as changes in amino groups, carboxyl groups, or side chains, caused by chemical reactions occurring in the process of bonding with crosslinkers to form peptide conjugates.

The peptide disclosed in the present specification may be a wild-type peptide identified and isolated from natural sources. On the other hand, the peptide disclosed in the present specification may be an artificial variant including an amino acid sequence in which one or more amino acids have been substituted, deleted, and/or inserted compared to a peptide consisting of an amino acid sequence selected from the group consisting of SEQ ID NOS: 1 to 8. Amino acid changes in wild-type polypeptides as well as in artificial variants include conservative amino acid substitutions that do not significantly affect the folding and/or activity of the protein. Examples of the conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine, valine and methionine), and aromatic amino acids (phenylalanine, tryptophan and tyrosine), and small amino acids (glycine, alanine, serine and threonine). Amino acid substitutions that generally do not alter specific activity are known in the art. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly, and as well as those in reverse.

Substantial modifications in the biological properties of the peptide are performed by selecting substituents that differ significantly in (a) their effect on maintaining the structure of the polypeptide backbone in a substituted region, for example, the sheet or helical conformation, (b) their effect on maintaining the charge or hydrophobicity of the molecule at a target site, or (c) their effect on maintaining the bulk of the side chain. Natural residues are divided into the following groups based on typical side chain characteristics:
(1) Hydrophobicity: norleucine, met, ala, val, leu, ile;
(2) Neutral hydrophilicity: cys, ser, thr;
(3) Acidicity: asp, glu;
(4) Basicity: asn, gln, his, lys, arg;
(5) Residues affecting chain orientation: gly, pro; and
(6) Aromatic: trp, tyr, phe.

Non-conservative substitutions will be made by exchanging a member of one of these classes with that of another class. Any cysteine residue unrelated to maintaining the proper conformation of the peptide may generally be substituted with serine to improve the oxidative stability of the molecule and prevent abnormal crosslinking. Conversely, cysteine bond(s) may be added to the peptide to improve its stability.

Another type of amino acid variant of the peptide is one in which the glycosylation pattern is altered. Altering means deleting one or more carbohydrate residues found in the peptide and/or adding one or more glycosylation sites that are not present in the peptide.

Glycosylation of peptides is typically N-linked or O-linked. N-linked refers to a carbohydrate residue attached to the side chain of an asparagine residue. The tripeptide sequences asparagine-X-serine and asparagine-X-threonine (where X is any amino acid except for proline) are recognition sequences for enzymatic attachment of carbohydrate residues to the asparagine side chain. Thus, the presence of one of these tripeptide sequences in the polypeptide creates a potential glycosylation site. O-linked glycosylation means attaching one of the sugars N-acetylgalactosamine, galactose and xylose to a hydroxy amino acid, most typically serine or threonine, but 5-hydroxyproline or 5-hydroxylysine may also be used.

The addition of glycosylation sites to peptides is conveniently accomplished by altering an amino acid sequence to contain one or more of the aforementioned tripeptide sequences (in the case of N-linked glycosylation sites). Such changes may be made by adding or substituting one or more serine or threonine residues to/in the initial antibody sequence (in the case of O-linked glycosylation sites).

Another aspect of the present invention provides a pharmaceutical composition for preventing or treating diabetes mellitus, including a peptide ("antidiabetic peptide") including an amino acid sequence selected from the group consisting of SEQ ID NOS: 1, 2, 3 and 5 as an active ingredient.

As a result of treating muscle cells with a peptide selected from SEQ ID NOS: 1 to 8, the present inventors confirmed that phosphorylation of Ampk and Akt, which promote sugar metabolism, increased, glucose tolerance was improved, and lipid metabolism was also improved.

AMP-activated protein kinase (AMPK) is an enzyme that plays an important role in maintaining cellular energy homeostasis, and is an AMP sensor that senses the ratio of AMP/ATP in cells. When ADP increases and AMP subsequently increases due to the consumption of ATP, AMP binds to the binding site of the γ subunit of AMPK, which exposes the catalytic domain of the α subunit. Thereafter, AMPKK, which is an upstream kinase of AMPK, phosphorylates threonine-172 to activate AMPK. AMPK promotes actions such as fatty acid oxidation and glucose influx.

Akt is a protein that is present in the intermediate process of insulin signaling, and promotes sugar metabolism when activated through phosphorylation.

Therefore, peptides selected from SEQ ID NOS: 1 to 8, which increase the phosphorylation of Ampk and Akt, may be usefully used for relieving/treating diabetes mellitus.

As used herein, the term "prevention" refers to all actions that suppress a disease or delay the onset of the disease by administering the pharmaceutical composition according to the present invention.

As used herein, the term "treatment" refers to all actions that ameliorate or beneficially change symptoms of a disease by administering the pharmaceutical composition according to the present invention.

According to an exemplary embodiment of the present invention, the peptide including the amino acid sequence of SEQ ID NO: 1 may be a peptide consisting of an amino acid sequence represented by SEQ ID NO: 1, 4, 6, 7, or 8.

Furthermore, the peptide including the amino acid sequence selected from the group consisting of SEQ ID NOS: 2, 3, and 5 may be a peptide consisting of an amino acid sequence selected from the group consisting of SEQ ID NOS: 2, 3, and 5, respectively.

Meanwhile, according to a specific exemplary embodiment of the present invention, the sequences of SEQ ID NO: 1 and SEQ ID NO: 3 have an antidiabetic activity. Therefore, the sequences of SEQ ID NOS: 10 and 12 to 14, including the sequence of SEQ ID NO: 1 or 3, may also have an antidiabetic activity.

According to an exemplary embodiment of the present invention, the pharmaceutical composition may include a peptide consisting of an amino acid sequence selected from the group consisting of SEQ ID NOS: 1 to 8, a peptide having a sequence homology of 80% or more with the amino acid sequence, or a peptide that is a fragment thereof.

The pharmaceutical composition according to an exemplary embodiment of the present invention may be applied to all animals including humans, dogs, chickens, pigs, cows, sheep, guinea pigs or monkeys.

The pharmaceutical composition according to an exemplary embodiment of the present invention may include additives such as a diluent, an excipient, a lubricant, a binder, a disintegrant, a buffer, a dispersant, a surfactant, a colorant, a flavoring agent or a sweetener, if necessary. The pharmaceutical composition according to an exemplary embodiment of the present invention may be prepared by a typical method in the art.

In the present invention, examples of a carrier, an excipient and a diluent which may be included in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

The pharmaceutical composition according to an exemplary embodiment of the present invention may be administered orally, rectally, transdermally, intravenously, intramuscularly, intraperitoneally, intramedullary, intrathecally, intradermally, or the like.

Formulations for oral administration may be tablets, pills, soft or hard capsules, granules, powders, liquids or emulsions, but are not limited thereto. Formulations for parenteral administration may be injections, drops, gels, suspensions, emulsions, suppositories, patches or sprays, but are not limited thereto.

The pharmaceutical compositions may be in the form of a sterile injectable preparation as a sterile injectable aqueous or oily suspension. The suspension may be formulated according to techniques known in the art using a suitable dispersant or wetting agent (for example, Tween 80) and a suspending agent. The sterile injectable preparation may also be a sterile injectable solution or suspension in a non-toxic parenterally acceptable diluent or solvent (for example, a solution in 1,3-butanediol). Examples of a vehicle and a solvent that may be acceptably used include mannitol, water, Ringer's solution, and an isotonic sodium chloride solution. Furthermore, sterile, non-volatile oils are typically used as a solvent or suspending medium. Any mild or non-volatile oil, including synthetic mono- or diglycerides, may be used for this purpose. Natural oils in which fatty acids such as oleic acid and glyceride derivatives thereof are pharmaceutically acceptable (for example, olive oil or castor oil), particularly polyoxyethylated versions thereof, are also useful in injectable preparations.

Parenteral administration of the pharmaceutical composition according to the present invention is particularly useful when the desired treatment involves sites or organs readily accessible by topical application. Examples of a carrier for topical administration of the composition of the present invention include, but are not limited to, mineral oil, liquid paraffin, white petrolatum, propylene glycol, a polyoxypropylene compound, emulsifying wax and water.

The active ingredient of the pharmaceutical composition according to the present invention may vary depending on the age, sex, body weight, pathological condition, and severity of a subject to be administered, administration route, or judgment of a prescriber. The determination of the dosage based on these factors is within the level of those skilled in the art, and the daily dose thereof may be, for example, 10 ng/kg/day to 10 mg/kg/day, specifically 0.1 µg/kg/day to 1mg/kg/day, more specifically 1 µg/kg/day to 100 µg/kg/day, and even more specifically 2 µg/kg/day to 50 µg/kg/day, but may be adjusted appropriately when different doses show different effects. The pharmaceutical composition according to an exemplary embodiment of the present invention may be administered once to three times a day, but is not limited thereto.

The terms used in the present specification are intended to describe specific embodiments only and are not intended to limit the present invention. Terms where the number before a noun is omitted are not intended to limit the quantity, but rather indicate the presence of one or more of the noun items mentioned. The terms "including," "having," and "containing" are to be interpreted as open terms (that is, meaning "including, but not limited thereto").

Referring to a range of values is an easy way to avoid individually mentioning each separate value within the range, and unless otherwise stated in the present specification, each separate value is incorporated in the present specification as if it is individually mentioned in the specification. All end values of the ranges are within the ranges and can be independently combined.

All the methods mentioned in the present specification may be performed in any suitable order unless otherwise indicated or clearly contradicted by the context. The use of any one embodiment and all embodiments or exemplary language (for example, "such as") is intended to more fully describe the present invention and is not intended to limit the scope of the present invention unless it is within the claims. No language in the specification should be interpreted as requiring any unclaimed element to be essential to the practice of the present invention. Unless otherwise defined, technical and scientific terms used in the present specification have the same meaning as commonly understood by a person with ordinary skill in the art to which the present invention pertains.

Still another aspect of the present invention provides a method for treating diabetes mellitus, the method including administering the pharmaceutical composition for preventing or treating diabetes mellitus to an individual in need thereof. The pharmaceutical composition and an administration method thereof are as described above.

Meanwhile, diabetic patients develop complications such as angina pectoris, myocardial infarction, diabetic retinopathy, and diabetic nephropathy due to long-term hyperglycemia. In particular, sarcopenia has recently been highlighted as a complication of diabetes mellitus (Lancet Diabetes Endocrinol 2013;1:106-14).

According to an exemplary embodiment of the present invention, the antidiabetic peptide has the activity of inhibiting an increase in glomerular size due to diabetes mellitus, protecting renal function, and inhibiting fat accumulation in muscle tissue. Therefore, the antidiabetic peptide of the present invention may be used as a pharmaceutical composition for preventing or treating diabetic complications, or a health functional food composition for preventing or relieving diabetic complications.

In the present invention, the diabetic complication may be selected from the group consisting of sarcopenia, diabetic nephropathy, diabetic neuropathy, diabetic myocardial infarction, diabetic retinopathy, diabetic cataracts, hyperlipidemia, fatty liver and diabetic foot ulcers.

According to an exemplary embodiment of the present invention, the diabetic complication may be sarcopenia or diabetic nephropathy.

Further, yet another aspect of the present invention provides a method for treating diabetic complications, the method including administering the pharmaceutical composition for preventing or treating diabetic complications to an individual in need thereof. The pharmaceutical composition and an administration method thereof are as described above.

### [Advantageous Effects]

The peptide according to the present invention improves sugar metabolism, improves insulin resistance, and inhibits amyotrophy while increasing muscle mass in diabetic and obese mouse models. In addition, the peptide according to the present invention shows an effect of treating and relieving diabetes mellitus and diabetic complications by regulating lipid metabolism and energy metabolism factors in muscle cells and adipocytes, inhibiting an increase in glomerular size caused by diabetes mellitus, protecting renal functions, and inhibiting fat accumulation in muscle tissue.

### [Description of Drawings]

FIG. 1 shows changes in Ampk phosphorylation observed after treating muscle cells (L6 skeletal muscle cell line) with antidiabetic peptides (Nos. 1 to 5 and 7 to 8).
FIG. 2 shows changes in Akt phosphorylation observed after treating muscle cells (L6 skeletal muscle cell line) with antidiabetic peptides (Nos. 1 to 8).
FIG. 3 shows changes in glucose uptake observed after treating muscle cells (C2C12 skeletal muscle cell line) with antidiabetic peptides (Nos. 1 to 8) and insulin (* p<0.05 compared with control).
FIG. 4 is a quantitative graph of (left) blood sugar change over time and (right) blood sugar change confirmed after administering an antidiabetic peptide (No. 8) to an obese mouse model (* p<0.05 compared with control).
FIG. 5 is the result showing the degree of glucose tolerance improvement using a quantitative graph after administering an antidiabetic peptide (No. 1) to an obese mouse model.
FIG. 6 confirms indicators related to sugar metabolism in the blood after administering antidiabetic peptides (Nos. 7 and 8) to a diabetic mouse model, and compares (A) glycated hemoglobin (HbA1c) levels, (B) degree of HbA1c change, (C) fasting blood glucose, (D) insulin levels, and (E) relative homeostatic model assessment for insulin resistance (HOMA-IR) values (* p<0.05 compared with control).
FIG. 7 confirms changes in muscle mass after administering antidiabetic peptides (Nos. 7 and 8) to a diabetic mouse model (* p<0.05 compared with control).
FIG. 8 shows changes in S6 phosphorylation observed after treating muscle cells (L6 skeletal muscle cell line) with antidiabetic peptides (Nos. 3, 6 and 7).
FIG. 9 shows changes in FoxO1 phosphorylation observed after treating muscle cells (L6 skeletal muscle cell line) with antidiabetic peptides (Nos. 1 to 8).
FIG. 10 shows changes in expression of PGC-1α and LPL genes, which improve lipid metabolism, observed after treating muscle cells (L6 skeletal muscle cell line) with antidiabetic peptides (Nos. 1, 3 and 8).
FIG. 11 shows changes in blood renin and creatinine after administering antidiabetic peptides (Nos. 7 and 8) to a diabetic mouse model (* p<0.05 compared with control).
FIG. 12 shows the results of staining fat in muscle tissue after administering antidiabetic peptides (Nos. 7 and 8) to a diabetic mouse model, and includes (left) a representative image of fat staining and (right) a graph quantifying the fat area (** p<0.01 compared with control).
FIG. 13 shows the results of confirming changes in glomerular size using histological staining after administering antidiabetic peptides (Nos. 7 and 8) to a diabetic mouse model, and includes (left) a representative image of glomerular staining and (right) a graph quantifying the glomerular area (** p<0.01 compared with control).

### [Modes of the Invention]

Hereinafter, one or more specific exemplary embodiments will be described in more detail through examples. However, these examples are provided only for exemplarily explaining the one or more specific exemplary embodiments, and the scope of the present invention is not limited to these examples.

### Peptide

The antidiabetic peptides discovered in the present invention are shown in the following Table 2.

**[Table 2]**

| | Peptide sequence | SEQ ID NO: |
|---|---|---|
| No. 1 | YFR | 1 |
| No. 2 | EQAEEER | 2 |
| No. 3 | REAGGAFGKR | 3 |
| No. 4 | EQAEEERYFR | 4 |
| No. 5 | AQSREQLAALKK | 5 |
| No. 6 | YFRAQSREQLAALKKHH | 6 |
| No. 7 | AGSIREAGGAFGKREQAEEERYFR | 7 |
| No. 8 | VDRGAGSIREAGGAFGKREQAEEERYFR | 8 |

### Example 1: Confirmation of activation of sugar metabolism-promoting signal pathway in muscle cells

An L6 skeletal muscle cell line was cultured in a 6-well plate and then starved with 0.5% FBS. Thereafter, the cell line was treated with an antidiabetic peptide at a concentration of 100 nM. After 1 hour, proteins were isolated by collecting the cells, and western blotting was performed to confirm whether Amp-activated protein kinase (Ampk) and Akt signal pathways were activated.

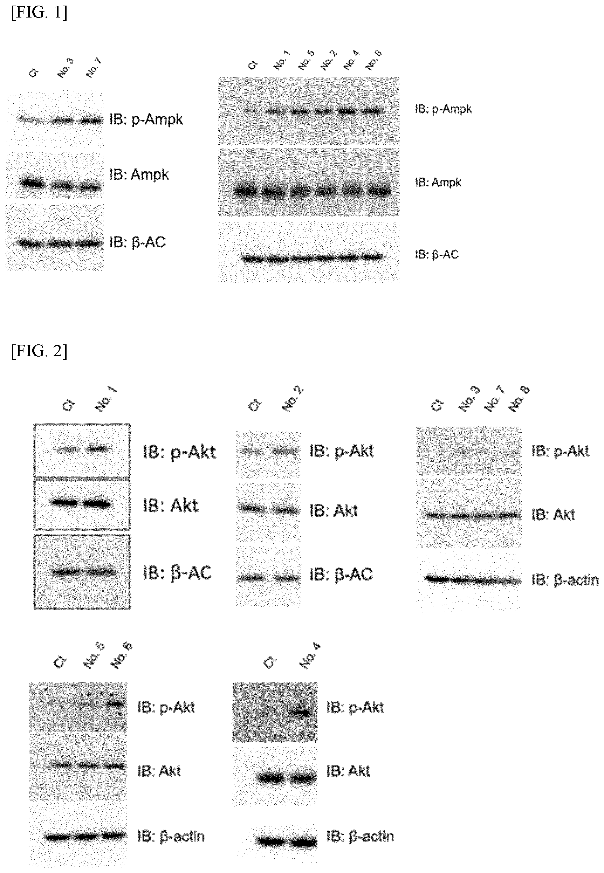

As a result, it could be confirmed that the degree of phosphorylation of both Apk (FIG. 1) and Akt (FIG. 2) was increased by antidiabetic peptide treatment (FIGS. 1 and 2). Through this, the sugar metabolism-promoting function of the antidiabetic peptide was confirmed.

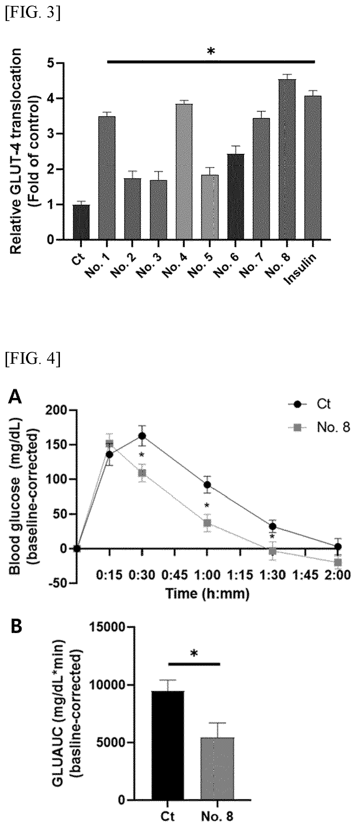

Similarly, in order to further verify the function of promoting glucose metabolism by antidiabetic peptide treatment, the degree of expression of a glucose transporter type 4 (GLUT4) glucose receptor, which plays an important role in glucose uptake, in the cell membrane was measured. It is known that GLUT4 moves from the cytoplasm to the cell membrane to promote sugar metabolism, and thus the expression level thereof increases. To confirm this, C2C12 skeletal muscle cells were starved with 0.5% FBS and then treated with the peptide and insulin, which is a positive control, at a concentration of 100 nM for 1 hour. Cells were fixed with 4% paraformaldehyde, incubated with a GLUT4-specific antibody at 37°C for 30 minutes, reacted with an o-phenylenediamine (OPD) solution, and absorbance was measured and compared with that of the control. As a result, the efficacy of antidiabetic peptides to increase glucose uptake was observed by observing significant GLUT4 translocation in all peptide-treated groups including insulin compared to the control (FIG. 3).

### Example 2: Confirmation of relieving impaired glucose tolerance

### 2-1. Construction of obese mouse model

5-week-old C57BL/6N male mice were obtained from Orient Bio, fed a normal diet, and used after an adaptation period of one week. The breeding environment was maintained at 18 to 24°C and a humidity of 50 to 60%, and the animals were given free access to feed and water during both the adaptation period and the experimental period. After the one-week adaptation period, the mice were induced to become obese through a high-fat diet to induce impaired glucose tolerance. The normal diet and high fat diet were prepared as shown in the following Table 3 and provided.

**[Table 3]**

| Normal diet (ND) and high fat diet (HFD) composition table | | |
|---|---|---|
| Ingredients | ND (g) | HFD (g) |
| Corn starch | 15 | 15 |
| Casein | 20 | 20 |
| Sucrose | 50 | 34 |
| Corn oil | 5 | 3 |
| Mineral mix | 3.5 | 3.5 |
| Vitamin mix | 1 | 1 |
| Cellulose | 5 | 5 |
| DL-methionine | 0.3 | 0.3 |
| Choline bitartrate | 0.2 | 0.2 |
| Lard | | 17 |
| Cholesterol | | 1 |
| BHT | 0.001 | 0.001 |
| Total | 100 (g) | 100 (g) |

### 2-2. Confirmation of whether glucose tolerance is improved

The mice in which obesity was induced were divided into a control and an experimental group. All mice were fasted for 12 hours, and PBS and a peptide (No. 1, 7, or 8; 2.5 mg/kg) were administered to the control and the experimental group, respectively. One hour later, a 20% glucose solution was intraperitoneally injected, and blood collected at each time period was analyzed using a blood glucose analyzer. As a result, in the experimental group to which the peptide (No. 8) was administered, an effect of significantly improving blood sugar metabolism was observed when correction was performed on the baseline compared to the control (left side of FIG. 4). Similarly, as a result of quantifying and comparing the degree of blood sugar change over time (GLUAUC) after baseline correction, a change in blood sugar was reduced by 31.1% by the peptide corresponding to SEQ ID NO: 7 compared to the control was observed, and through this result, it can be seen that the antidiabetic peptide improves glucose tolerance.

### Example 3: Confirmation of antidiabetic efficacy (in vivo)

### 3-1. Construction of diabetic mouse model

It was confirmed whether the antidiabetic peptide relieves diabetes mellitus induced by leptin receptor defects. As an animal model, 5-week-old C57BLKS/J-db/db male mice were provided by Central Lab. Animal Inc., fed a normal diet, and used after an adaptation period of one week. The breeding environment was maintained at 18 to 24°C and a humidity of 50 to 60%, and the animals were given free access to normal feed and water during both the adaptation period and the experimental period. Mice that completed the adaptation period were divided into a control (intraperitoneal injection) and a peptide administration group. PBS and antidiabetic peptides (Nos. 7 and 8; 2.5 mg/kg) were intraperitoneally administered to the control and peptide administration groups, respectively, for a total of 8 weeks.

### 3-2. Confirmation of antidiabetic efficacy (tissue examination)

After 8 weeks, various tests were performed on the diabetic mouse model.

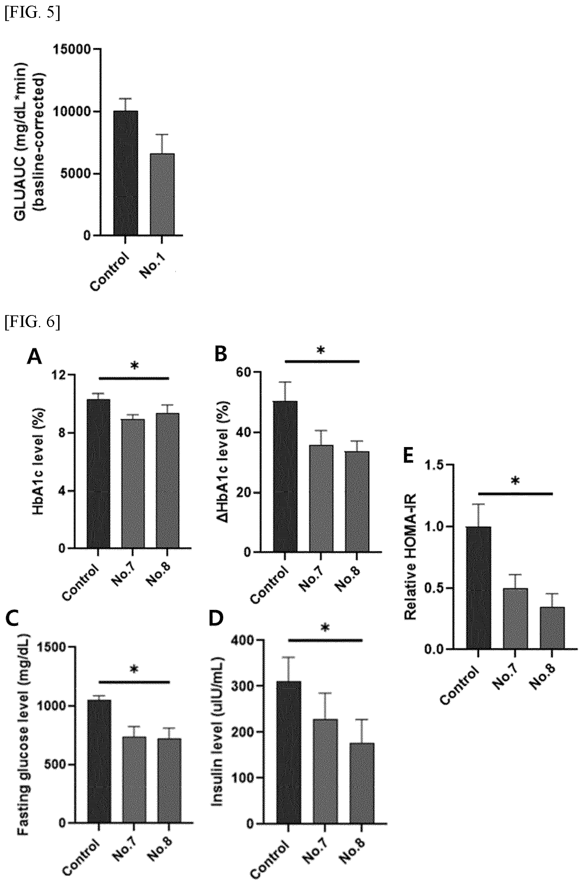

A blood examination was performed to confirm indicators related to sugar metabolism in the blood. As a result of measuring the glycated hemoglobin (HbA1c) levels and the amount of change, it could be confirmed that the glycated hemoglobin level significantly decreased in the peptide administration group (FIGS. 6A and 6B), and it was observed that fasting blood sugar and insulin levels were also lowered (FIGS. 6C and 6D). Furthermore, as a result of confirming the level of the homeostatic model assessment for insulin resistance (HOMA-IR), which is the corresponding indicator in order to confirm whether insulin resistance induced by diabetes mellitus was improved, it was confirmed that the level was reduced by peptide treatment (FIG. 6E). Through the results shown in FIG. 6, it could be seen that antidiabetic peptides generally improve blood indicators related to diabetes mellitus.

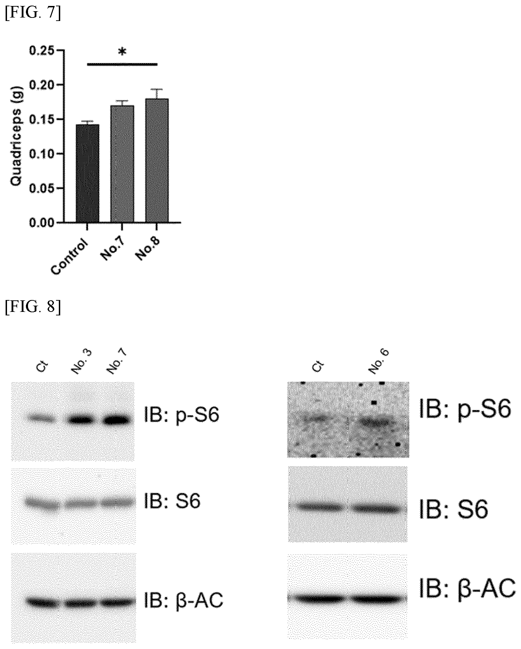

Meanwhile, the progression of diabetes mellitus brings about various complications. Muscle tissue is an organ that plays the most important role in sugar metabolism in the human body, and as diabetes mellitus progresses, muscle loss or weakened muscle function may appear. Thus, as a result of observing changes in muscle tissue, it could be seen that muscle mass (quadriceps) increased due to peptide treatment (FIG. 7).

### Example 4: Confirmation of antidiabetic efficacy (in vitro)

### 4-1. Verification of muscle biosynthesis and amyotrophy inhibitory efficacy

L6 skeletal muscle cells were cultured in a 6-well plate and then starved with 0.5% FBS. Thereafter, the cells were treated with an antidiabetic peptide at a concentration of 100 nM for 1 hour, proteins were collected, and then western blotting was performed.

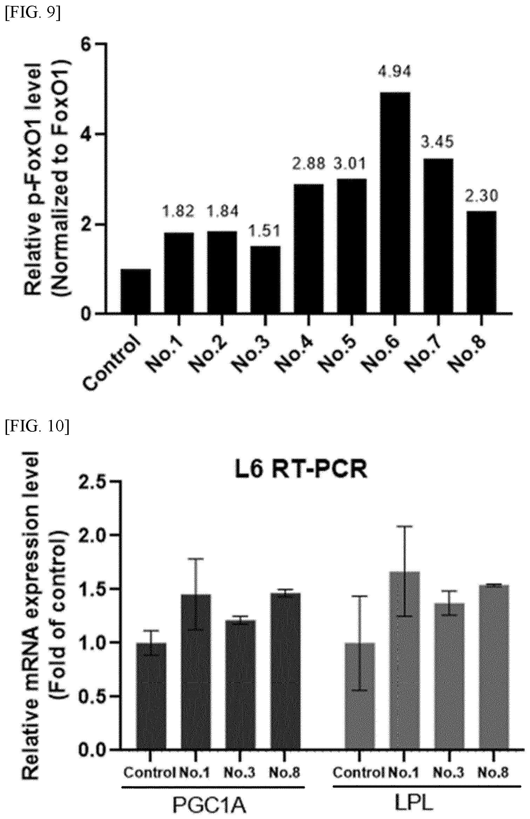

As a result of performing western blotting, the efficacy of relieving muscle loss by promoting muscle biosynthesis and inhibiting amyotrophy may be confirmed by observing that the phosphorylation of FoxO1 (FIG. 9) protein, which exhibits an amyotrophy-inhibiting function, along with S6 (mTOR marker) (Nos. 3, 6, and 7; FIG. 8), which is known to activate the cell signaling pathway as a main indicator of muscle biosynthesis, is increased by the peptides. Specifically, the phosphorylation of S6 protein was increased 188% by peptide No. 1, 365% by peptide No. 2, 224% by peptide No. 4, 494% by peptide No. 5, and 414% by peptide No. 8. Further, peptide No. 1 increased the phosphorylation of FoxO1 and simultaneously also decreased the expression level of total FoxO1.

### 4-2. Confirmation of expression of genes related to lipid metabolism

After muscle cells were treated with antidiabetic peptides (Nos. 1, 3, and 8), changes in the expression of peroxisome proliferator-activated receptor gamma coactivator 1-alpha (PGC-1α), an indicator of energy metabolism activation, lipid metabolism activation, and glucose metabolism improvement, and lipoprotein lipase (LPL) genes were confirmed by RT-PCR.

As a result of confirmation, it could be seen that the expression of PGC-1α and LPL genes increased due to antidiabetic peptide treatment (FIG. 10).

### Example 5: Example 5: Verification of effects of relieving diabetic complications

Various tissue examinations were performed on a db/db diabetic mouse model to confirm the efficacy of relieving diabetic complications. First, in order to confirm muscle loss-related effects, RNA was isolated from muscle tissue and the expression levels of muscle loss-related genes were confirmed. As a result, it was confirmed that treatment with peptide No. 7 or 8 decreased the expression of adipocyte protein 2 (AP2) and CCAAT enhancer binding protein α (C/EBPα) genes, which are associated with lipid accumulation, and increased the expression of the peroxisome proliferator-activated receptor α (PPARα) gene, which is associated with fatty acid oxidation. Further, the effect of relieving muscle loss was further verified by confirming a decrease in the muscle RING Finger-1 (Murf-1) gene, which contributes to muscle loss and amyotrophy (Table 4).

**[Table 4]**

| Item / Material | No. 7 | No. 8 |
|---|---|---|
| AP2 | -52.5 % | -54.7 % |
| PPARα | +94.1 % | +12.9 % |
| C/EBPα | -18.6 % | -45.5 % |
| Murf-1 | -90.1 % | -36.0 % |

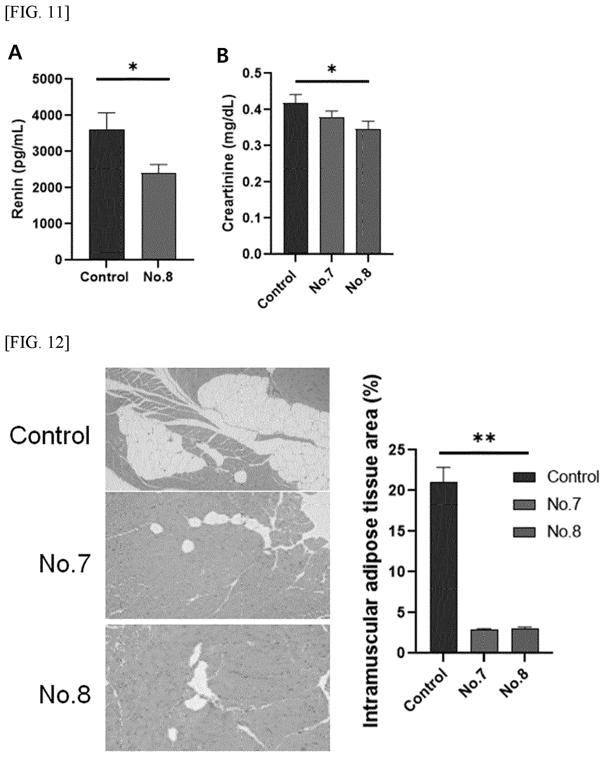

Blood tests observed renin and creatinine, which are excreted as renal function weakens, and confirmed that the levels of renin and creatinine were significantly reduced by antidiabetic peptide treatment (FIG. 11).

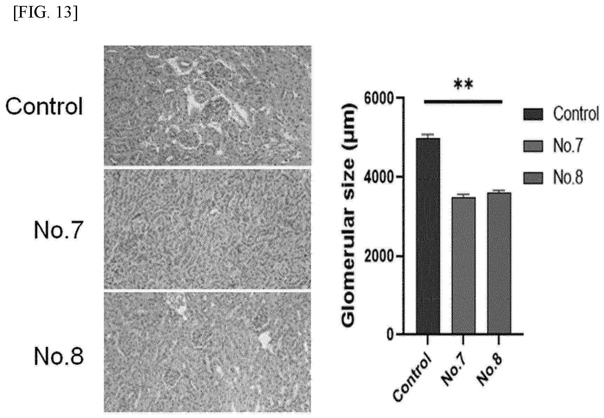

H&E tissue staining was performed to confirm the final tissue pathology. As a result, it was confirmed that lipid accumulation in muscle tissue accompanying diabetes mellitus was improved by antidiabetic peptide treatment (FIG. 12, left side), and furthermore, the improvement in lipid accumulation in muscle tissue was quantitatively significant (FIG. 12, right side). In addition, it was confirmed that antidiabetic peptide treatment inhibited an increase in size caused by intrarenal glomerular overload (FIG. 13, left side) and quantitatively significantly reduced the size (FIG. 13, right side). Through the results of the present example, the effect of antidiabetic peptides in relieving diabetic complications was verified.

## Claims

1. A peptide consisting of an amino acid sequence selected from the group consisting of SEQ ID NOS: 1 to 14.

2. A polynucleotide encoding the peptide of claim 1.

3. A pharmaceutical composition for use in preventing or treating diabetes mellitus, comprising a peptide comprising an amino acid sequence selected from the group consisting of SEQ ID NOS: 1, 2, 3 and 5 as an active ingredient.

4. The pharmaceutical composition for use of claim 3, wherein the peptide comprising the amino acid sequence represented by SEQ ID NO: 1 is a peptide consisting of an amino acid sequence represented by SEQ ID NO: 1, 4, 6, 7, or 8.

5. The pharmaceutical composition for use of claim 3, wherein the peptide improves insulin resistance.

6. The pharmaceutical composition for use of claim 3, wherein the peptide promotes sugar metabolism.

7. The pharmaceutical composition for use of claim 3, wherein the peptide promotes glucose uptake.

8. The pharmaceutical composition for use of claim 3, wherein the peptide promotes muscle biosynthesis or inhibits amyotrophy.

9. A method for treating diabetes mellitus, the method comprising administering the pharmaceutical composition for preventing or treating diabetes mellitus according to claim 1 to an individual in need thereof.

10. A pharmaceutical composition for use in preventing or treating diabetic complications, comprising an amino acid sequence selected from the group consisting of SEQ ID NOS: 1, 2, 3 and 5 as an active ingredient.

11. The pharmaceutical composition for use of claim 10, wherein the peptide comprising the amino acid sequence represented by SEQ ID NO: 1 is a peptide consisting of an amino acid sequence represented by SEQ ID NO: 1, 4, 6, 7, or 8.

12. The pharmaceutical composition for use of claim 10, wherein the diabetic complication is selected from the group consisting of sarcopenia, diabetic nephropathy, diabetic neuropathy, diabetic myocardial infarction, diabetic retinopathy, diabetic cataracts, hyperlipidemia, fatty liver and diabetic foot ulcers.

13. A method for treating diabetic complications, the method comprising administering the pharmaceutical composition for preventing or treating diabetes mellitus according to claim 10 to an individual in need thereof.
